# EUROPEAN PATENT APPLICATION

(11) **EP 1 677 110 A1**
(43) Date of publication of application: **05.07.2006**
(21) Application number: 04773565.9
(22) Date of filing: 27.09.2004
(51) Int. Cl.: G01N 33/53, G01N 37/00, C12M 1/00

(54) **METHOD FOR PRODUCING BIOASSAY SUBSTRATE BY SUPERPOSING TWO SUBSTRATES ONE ON ANOTHER AND BIOASSAY SUBSTRATE**

(30) Priority: 03.10.2003 JP 2003346277
(71) Applicant: SONY CORPORATION, Tokyo 141-0001 (JP)
(72) Inventor: OHNISHI, Michihiro, Shinagawa-ku, Tokyo 141 (JP); AKI, Yuichi, Shinagawa-ku, Tokyo 141 (JP); INAGAKI, Minoru, Shinagawa-ku, Tokyo 141 (JP)
(74) Representative: Horner, David Richard
(86) International application number: PCT/JP2004/014536
(87) International publication number: WO 2005/033703

(57) **Abstract**

A bioassay plate is provided with two pairs of opposing electrodes in a reaction region, and by imposing a predetermined electric field on the reaction region, the bioassay plate makes it possible to perform high-order structural adjustment, migration, immobilization and the like of a substance as desired. A first substrate (11) is provided with a detection well (X), which is in turn equipped at least with a reaction region (R) for providing a place of interaction between the substances and also with a first electrode (E₁₁) arranged facing the reaction region (R). A second substrate (12) is provided at least with a second electrode (E₁₂) which can impose an electric field on the reaction region (R) in association with the first electrode (E₁₁). The present invention provides a bioassay plate (1) formed of these two substrates (11), (12) stacked together such that the first electrode (E₁₁) and the second electrode (E₁₂) are located opposite to each other, and also a production method of the plate (1).

## Description

### Technical Field

This invention relates to a technology on DNA chips and other bioassay plates. More specifically, this invention is concerned with a bioassay plate with opposing electrodes formed by aligning and stacking two electrode-carrying substrates together and also with a production method of the bioassay plate.

### Background Art

A description will be made of primary background technologies relating to the present invention. A first background technology (conventional technology) is the technology on integrated plates for bioassay, which are generally called "DNA chips" or "DNA microarrays" (hereinafter collectively called "DNA chips") (see, for example, Japanese Translations of PCT for Patent No. Hei 4-505763 and Japanese Translations of PCT for Patent No. Hei 10-503841).

This DNA chip technology is characterized in that a comprehensive analysis of intermolecular reactions such as hybridization can be performed, because a variety of numerous DNA oligonucleotide chains, cDNAs (complementary DNAs) or the like re integrated on a glass substrate or silicone substrate. Accordingly, DNA chips are used for the mutation analysis of genes, SNPs (single-base polymorphisms) analysis, gene expression frequency analysis, and the like, and have begun to find utility in a wide range of fields such as drug developments, pharmacogenomics and forensic medicine. In addition DNA chips, protein chips with proteins immobilized on substrates, biosensor tubes for analyzing interaction between various substances, and the like have also been developed.

A second background technology is the technology that pertains to the action of an electric field on a substance existing in a charged form in a liquid phase. Described specifically, a nucleotide chain (nucleic acid molecule) is known to extend or migrate when subjected to the action of an electric field in a liquid phase. As its principle, an ionic atmosphere is considered to be formed by phosphate ions (negative charges), which make up the skeleton of a nucleotide chain, and hydrogen atoms (positive charges) formed as a result of ionization of water existing around the phosphate ions. Polarization vectors (dipoles) produced by these negative charges and positive charges are oriented in a single direction as a whole upon imposition of a high-frequency high voltage. As a result, the nucleotide chain extends and, when a non-uniform electric field in which electric lines of force locally concentrate is imposed, the nucleotide chain migrates toward a position at which electric lines of force concentrate (see Seiichi Suzuki, Takeshi Yamanashi, Shin-ichi Tazawa, Osamu Kurosawa and Masao Washizu: "Quantitative analysis on electrostatic orientation of DNA in stationary AC electric field using fluorescence anisotropy", IEEE Transaction on Industrial Applications, Volume 34, No.1, PP 75 to 83 (1998)). This migration is called "dielectrophoresis". When a DNA solution is placed between microelectrodes having, for example, a gap of from several tens to several hundreds µm and a high-frequency electric field of 1 MV/m and 1 MHz or so is imposed on the DNA solution, DNA which exists in the form of a random coil undergoes dielectric polarization, and as a result, the DNA molecule is stretched linearly in parallel with the electric field. It is known that under the electrodynamic effect called "dielectrophoresis", the polarized DNA is spontaneously attracted to an electrode end and is immobilized with an end thereof kept in contact with an electrode edge (see, Masao Washizu: "DNA Handling To Be Performed under Watching", Journal of Visualization Society of Japan", Volume 20, No.76 (January 2000).

According to the above-described DNA chip technology, a reaction region is set beforehand on a plate to provide a place of interaction between substances, and detection nucleotide chains such as a DNA probe are immobilized in the reaction region to permit an analysis of hybridization as an interaction between the detection nucleotide chains and complementary target nucleotide chains. The DNA chip technology is, however, accompanied by serious technical problems in that the reaction takes long time due to the low efficiency of hybridization and the accuracy of detection is low for the occurrence of false positive and false negative.

Upon practicing this DNA chip technology, problems associated with a steric hindrance caused by the random-coiled, high-order molecule structure and an interference (for example, adhesion and contact) of the detection nucleotide chains with the surrounding surface can be eliminated and as a consequence, the efficiency of hybridization can be improved, provided that the detection nucleotide chains existing with their end positions immobilized in the reaction region can be controlled in an extended form.

Principal objects of the present invention are, therefore, to provide a bioassay plate which permits performing high-order structural adjustment, migration, immobilization and the like of a substance as desired by imposing a predetermined electric field on a reaction region provided with opposing electrodes, and also to provide a method for its production.

### Disclosure of Invention

The basic construction of a bioassay plate provided by the present invention has the form that a "first substrate", which is provided at least with a reaction region for providing a place of an interaction between substances and also with a first electrode arranged to face the reaction region, and a "second substrate", which is provided at least with a second electrode formed to be located oppose the first electrode, are stacked together. Further, an axis of opposition between the first electrode and the second electrode may be perpendicular to a bottom wall of the reaction region.

It is also possible to provide a bioassay plate of the construction that a light source is arranged at a position of the first electrode and a light-receiving portion is arranged at a position of the second electrode, or of the construction that a light-receiving portion is arranged at a position of the first electrode and a light source is arranged at a position of the second electrode.

The present invention also provides a method for producing a bioassay plate, which incluldes providing a "first substrate" having a detecting portion provided at least with a reaction region for providing a place of an interaction between substances and also with a first electrode arranged to face the reaction region and a "second substrate" provided at least with a second electrode which permits imposition of an electric field to the reaction region in association with the first electrode; and stacking these two substrates together such that the first electrode and the second electrode are located opposite each other.

The present invention also provides a production method, which incluldes stacking a "first substrate", which carries thereon an array of plural detecting portions as defined above, and a "second substrate", which is provided with a common second electrode for establishing a relation of opposing electrodes with first electrodes as defined above and arranged in the respective plural detecting portions as a unit, together. This "common second electrode" can simplify the electrode construction as it makes it possible to form opposing electrodes between itself and the plural first electrodes.

In the case of stacking two disk-shaped substrates together, the present invention also provides a production method which incluldes stacking a disk-shaped "first substrate", which carries thereon plural detecting portions as defined above and arranged in the form of any one of radial arrays, concentric arrays or spiral arrays from a center of the substrate, and a "second substrate", which is provided with a strip-shaped or line-shaped, common second electrode as defined above and formed extending over some or all of the detecting portions belonging to one of the arrays, together.

Definitions of certain main technical terms used in the present invention will now be described. The term "interaction" as used in the present invention has a broad meaning and includes chemical bondings between substances, including non-covalent bonding, covalent bonding and hydrogen bonding, or dissociations, and embraces therein, for example, hybridization as complementary bonding between nucleic acids (nucleotide chains).

Next, the term "opposing electrodes" means at least a pair of electrodes, which are arranged with their electrode surfaces opposing against each other. The term "axis of opposition" means an axis formed by a straight line that connects the centers of the opposing two electrode surfaces with each other.

In the present invention, the term "nucleic acid" means a polymer of the phosphoric acid ester of a nucleoside in which a purine or pyrimidine base and a saccharide radical are linked together via a glycoside bond. The term "nucleic acid", therefore, has a broad meaning and encompasses therein oligonucleotides including DNA probes, polynucleotides, DNAs (full lengths or their fragments) formed by polymerization of purine nucleotide or pyrimidine nucleotide, cDNAs (cDNA probes) obtained by reverse transcription, RNAs, polyamide nucleotide derivatives (PNAs), and the like.

The term "hybridization" means a complementary chain (double-stranded) forming reaction between nucleotide chains equipped with complementary base sequence structures. The term "mishybridization" means a complementary chain forming reaction which is as defined above and is not normal, and is often abbreviated as "mishybri".

The term "reaction region" means a region that can provide a place of reaction for hybridization or other interaction. Illustrative is a place of reaction, which has the shape of a well and can store a liquid phase, gel or the like therein. An interaction which can be performed in such a reaction region shall not be narrowly limited insofar as it conforms with the objects and advantageous effects of the present invention. For example, further to an interaction between single-stranded nucleic acids, that is, hybridization, it is possible to form a desired double-stranded nucleic acid from a detection nucleic acid and then to conduct an interaction, enzyme response reaction or other intermolecular reaction between the double-stranded nucleic acid and a peptide (or protein). Use of the above-described double-stranded nucleic acid can analyze, for example, the bond or the like between a receptor molecule, such as a hormone receptor, as a transcription factor and a DNA fragment of a reactive sequence.

The term "steric hindrance" means a phenomenon that a desired reaction (hybridization in the present invention) becomes more difficult to take place as a result of the difficulty of a counterpart reactant molecule in approaching the center or the like of a reaction in a reactant molecule due to the existence of a bulky substituent group in the proximity of the center or the like of the reaction in the reactant molecule or the conformation or stereostructure (high-order structure) of the reactant molecule.

The term "dielectrophoresis" means a phenomenon that in a field where the electric field is not uniform, molecules are driven toward a location where the electric field is strong. When an alternating voltage is applied, similar driving effect can be obtained as in the case of a direct current because the polarity of polarization is also reversed as the polarity of the applied voltage is reversed (See, Teru Hayashi, Editorial Supervisor: "Micromachines and Materials Engineering", PP 37 to 46, Chapter 5 "Cell and DNA Manipulation" (CMC Publishing Co.)).

The term "bioassay plate" means an information-integrated plate useful for a biochemical or molecular biological analysis or study, and embraces therein so-called DNA chips.

According to the present invention, a bioassay plate provided with opposing electrodes can be produced at low cost and with ease by making use of the optical disk production technology which is widely used these days and has been established. The imposition of a predetermined electric field at a predetermined timing across the opposing electrodes of the bioassay plate makes it possible to perform a high-order structural adjustment of a substance such as a nucleic acid existing in the reaction region, migration of the substance along the electric field, immobilization of the substance at end positions of its molecules, and the like as desired.

### Brief Description of Drawings

FIG. 1 is a view for describing one example of a basic construction of a bioassay plate according to the present invention and a basic production method of the bioassay plate (1).
FIG. 2 is a cross-sectional view taken in the direction of arrows I-I in FIG. 1 (in a stacked form).
FIG. 3 is a view for describing one example of a second basic construction of a bioassay plate according to the present invention and a basic production method of the bioassay plate (2).
FIG. 4 is a cross-sectional view taken in the direction of arrows II-II in FIG. 3 (in a stacked form).
FIG. 5 is a view for describing a third basic construction of a bioassay plate according to the present invention, and is a view showing the construction of a disk-shaped first substrate (31) constituting the bioassay plate (3).
FIG. 6 is a view showing the construction of a disk-shaped second substrate (32) constituting the bioassay plate (3).
FIG. 7 is a cross-sectional view of the bioassay plate (3) with the first substrate (31) and the second substrate (32) stacked together.
FIG. 8 is a view illustrating the construction of a modification (41) of the disk-shaped first substrate which can be adopted in the bioassay substrate according to the present invention.
FIG. 9 is a view depicting another embodiment which can be adopted when forming opposing electrodes in the bioassay plate according to the present invention.
FIG. 10 is a view for describing one example of a developed form of the bioassay substrate (1) according to the present invention.
FIG. 11 is an external perspective view of an essential part of the bioassay substrate (1).
FIG. 12 is a vertical cross-sectional view of the essential part, and is a view schematically illustrating a state that a target DNA (T) is migrating while being extended under an electric field.
FIG. 13 is a vertical cross-sectional view of the essential part, and is a view schematically illustrating a state that hybridization has proceeded between a DNA probe (D) and the target DNA (T) to form a double stranded DNA.

### Best Modes for Carrying out the Invention

With reference to the accompanying drawings, a description will hereinafter be made about a preferred manner for practicing the method according to the present invention for the production of a bioassay plate and also about certain preferred embodiments of the bioassay plate.

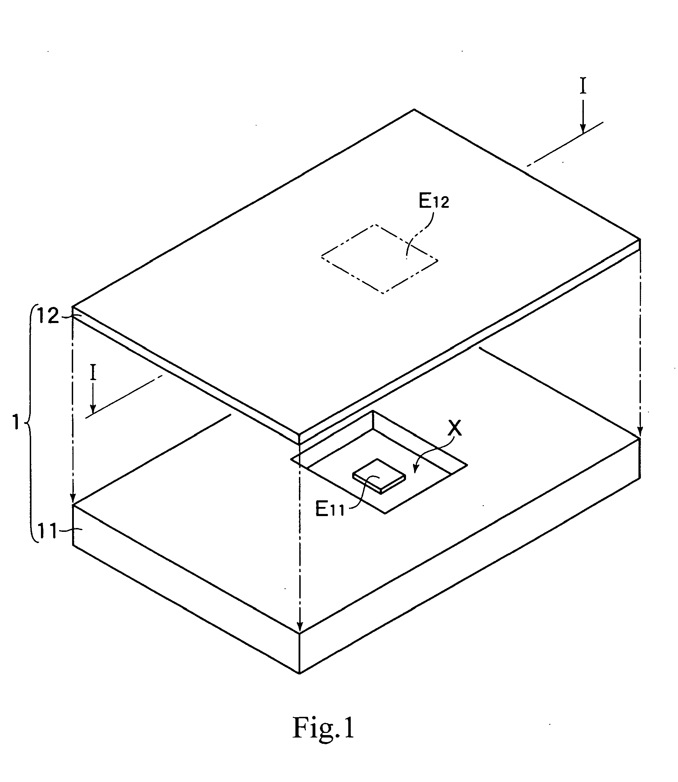

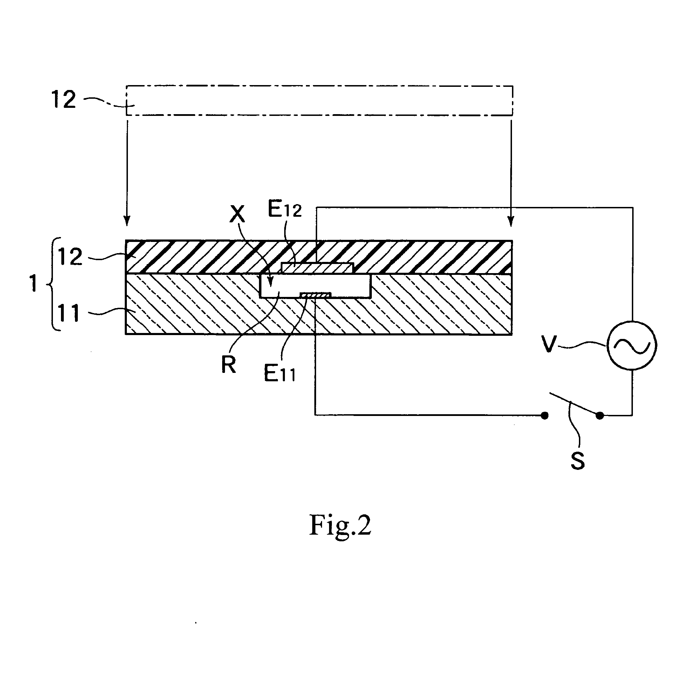

Firstly, FIG. 1 is a view for describing one example of a basic construction of a bioassay plate according to the present invention and a basic production method of the bioassay plate, and FIG. 2 is a cross-sectional view taken in the direction of arrows I-I in FIG. 1. Numeral 1 shown in these FIG. 1 and FIG. 2 indicates the bioassay plate according to the present invention.

The bioassay plate 1 is composed of a lower-side, first base plate designated at numeral 11 and an upper-side second substrate indicated at numeral 12. This bioassay substrate 1 is produced by accurately aligning and stacking these two substrates 11, 12 with each other.

The first substrate 11 and second substrate 12 are formed of glass or synthetic resin. It is desired to form at least the substrate, onto which excitation light for use in detecting an interaction such as hybridization is irradiated, with a material which is transparent in the predetermined wave length range of the excitation light. In this embodiment, the first substrate 11 is formed of a transparent resin.

At a predetermined position (for example, the central position) of the lower-side, first substrate 11, at least one detecting portion X is arranged. This detecting portion X is provided with a reaction region R and a first electrode E₁₁. The reaction region is a well (recess) opening upwards and having a predetermined volume. The first electrode E₁₁ is arranged centrally on a bottom wall of the reaction region R, and has been formed with an electrically-conductive material such as a metal like gold or aluminum or light-transmitting ITO (indium tin oxide) .

The reaction region R functions as a region or space, which can store a solution or hold a gel or the like as a place of an interaction. When the second substrate 12 has been accurately aligned with and stacked on the first substrate 1, the first electrode E₁₁ arranged in the reaction region R forms opposing electrodes, which are equipped with an axis of opposition in a direction perpendicular to the bottom wall of the reaction region R, in association with a second substrate E₁₂ arranged at a predetermined position of the second substrate 12. These electrodes E₁₁, E₁₂ play a role to form an electric field in the medium within the reaction region R when a voltage is imposed across both of the electrodes E₁₁-E₁₂₋It is to be noted that the distance between the opposing electrodes can be from 1 µm to 1 mm or so.

It is also possible to make the first electrode E₁₁ function as a detection surface for the immobilization of a detecting substance represented by the DNA probe D. In this case, surface treatment is applied beforehand to the first electrode E₁₁ such that molecule ends of a detecting substance such as the DNA probe D can be immobilized. It is to be noted that the second electrode E₁₂ can also be used as an immobilizing detection surface.

Taking as an example the case that the detecting substance is the DNA probe D, it is possible, as its immobilizing method, to immobilize the DNA probe D. through a reaction such as a coupling reaction between the surface of the electrode E₁₁ and molecule ends of the DNA probe D. For example, an electrode surface subjected to surface treatment with streptoavidin is suited for the immobilization of molecule ends of a biotinylated DNA probe D. An electrode surface subjected to surface treatment with thiol (SH) groups, on the other hand, is suited for the immobilization of a DNA probe D, which has been end-modified with thiol groups, via disulfide bonds (-S-S- bonds).

It is to be noted that the surfaces of the first electrode E₁₁ of the first substrate 11 and the second electrode E₁₂ of the second substrate 12 may desirably be covered with insulating layers (not shown) formed with a material selected from SiO₂, SiN, SiOC, SiOF, SiC or TiO₂ (this will apply equally to the detecting portions of the other embodiment to be described subsequently; its description will be omitted hereinafter). This is to avoid an electrochemical reaction with an ionic solution which may be stored in the reaction region R in some instances.

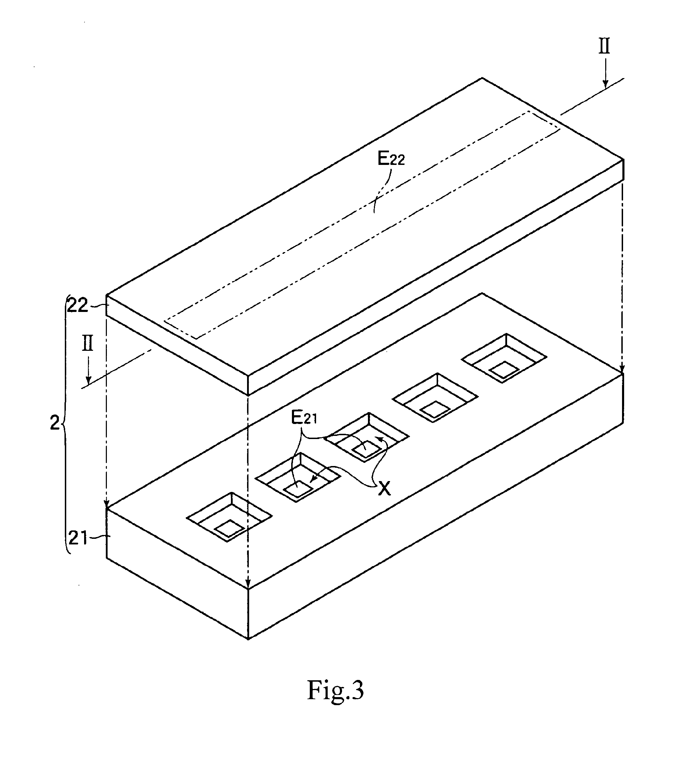

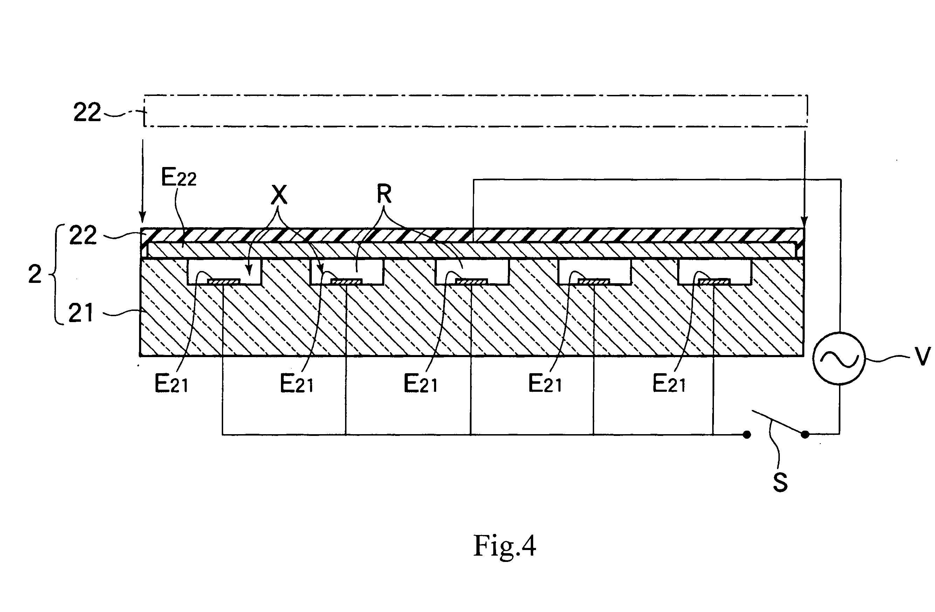

FIG. 3 is a view for describing one example of a second basic construction of a bioassay plate according to the present invention and a basic production method of the bioassay plate, and FIG. 4 is a cross-sectional view taken in the direction of arrows II-II in FIG. 3.

The bioassay plate indicated at numeral 2 in FIG. 3 and FIG. 4 is produced by stacking two substrates together as in the case of the above-described bioassay plate 1. Specifically, the bioassay plate 2 is produced by stacking together a first substrate indicated at numeral 21 and a second substrate designated at numeral 22.

In the lower-side, first substrate 21 which constitutes the bioassay plate 2, detecting portions X as many as 5 in total (not limited to 5) are arranged side by side at predetermined intervals, and first electrodes E₂₁ are arranged on bottom walls of the reaction regions R in the respective detecting portions X. The other substrate, i.e., the second substrate 22 is provided with a line-shaped or strip-shaped, second electrode E₂₂ which extends in the direction of the length of the substrate. It is to be noted that the first electrodes E₂₁ may be in the form of a line-shaped or strip-shaped, unitary electrode like the second electrode E₂₂ instead of such discrete independent electrodes as shown in the drawing.

When the second substrate 22 is stacked over the first substrate 21, the line-shaped or strip-shaped, second electrode E₂₂ functions as a common electrode which is located opposite all the first electrodes E₂₁. In other words, these electrodes are designed such that, when a switch S is turned on, electric fields can be concurrently formed in media within the individual reaction regions R via a power supply V (see FIG. 4).

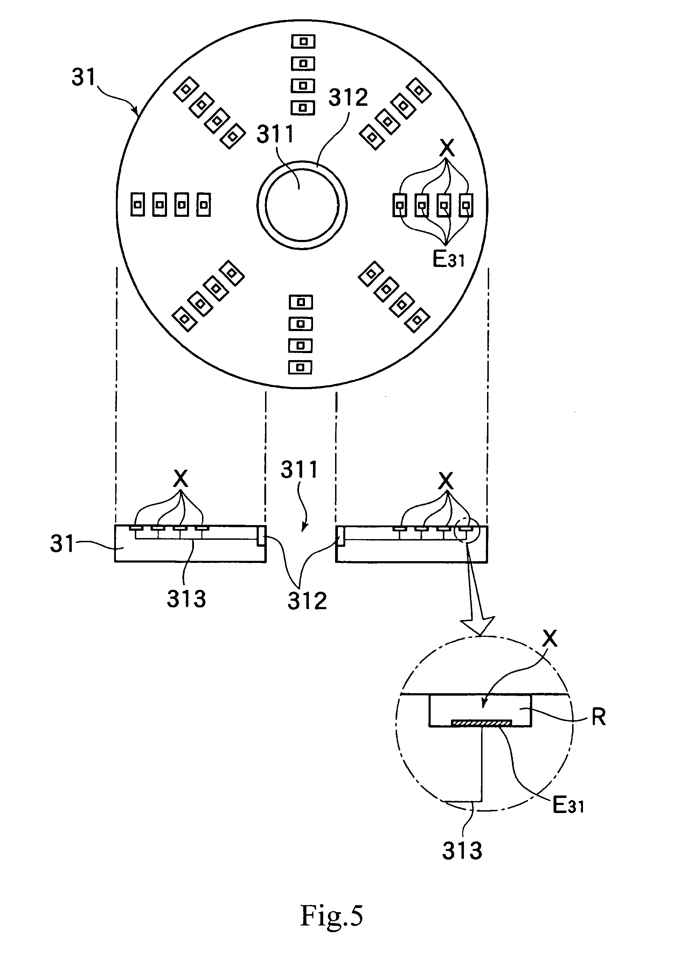

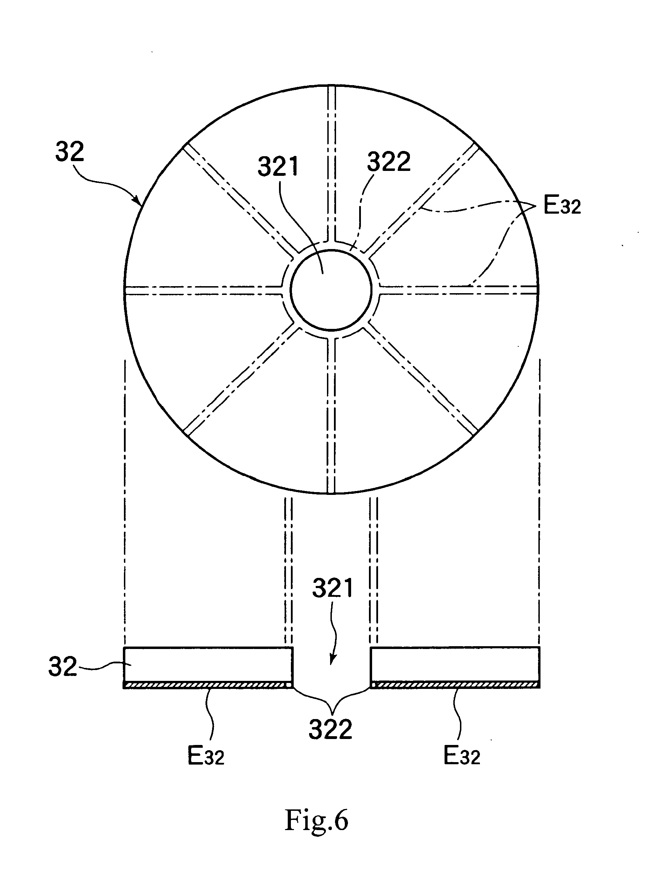

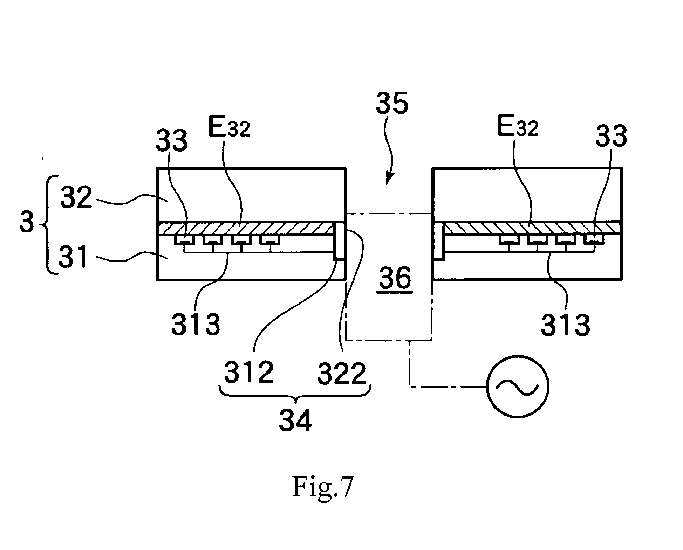

FIG. 5 is a view for describing a third basic construction of a bioassay plate according to the present invention, and is a view showing the construction of a disk-shaped first substrate constituting the bioassay plate. FIG. 6 is a view showing the construction of a disk-shaped second substrate constituting the bioassay plate. FIG. 7 is a cross-sectional view of the bioassay plate with the first substrate and the second substrate stacked together.

The disk-shaped first substrate 31 shown in FIG. 5 is provided with a circular hole 311 formed at its central part, and on an upper surface of the first substrate 31, a group of detecting portions X are arranged in eight arrays in total such that the detecting portions form a radial or concentric pattern. First electrodes E₃₁ are arranged in the respective detecting portions X (specifically, their reaction regions R). This group of first electrodes E₃₁ are connected to a wiring 313 which leads to a ring-shaped first current-carrying portion 312 formed around the hole 311. It is to be noted that the first current-carrying portion 312 is exposed on a peripheral wall of the hole 311.

In the disk-shaped second substrate 32 depicted in FIG. 6 and having the same diameter as the above-described first substrate 31, a hole 321 of the same diameter as the hole 311 is formed at is central part. On the lower surface of the second substrate 32, a common electrode E₃₂ is formed as lines or strips arranged as many as eight in total (not limited to eight) to create a radial pattern. The common electrode E₃₂ is connected to a ring-shaped, second current-carrying portion 322 formed around the hole 321. It is to be noted that the first current-carrying portion 322 is exposed on a peripheral wall of the hole 321.

When the bioassay plate 3 is formed by accurately subjecting these first substrate 31 and second substrate 32 to predetermined positioning and stacking them together in accordance with a known optical disk bonding technology or the like, the common electrode E₃₂ of the second substrate 32 are accurately arranged right above the individual arrays of the first electrodes E₃₁ located on the lower side such that the common electrode E₃₂ transversely or longitudinally extends over the respective reaction regions R (see FIG. 7).

At this time, the first current-carrying portion 312 and the second current-carrying portion 322 are integrated to form a single current-carrying portion as illustrated at numeral 34 in FIG. 7. A current-carrying element 36 is inserted in a hole 35 to come into contact with this current-carrying portion 34, so that a current is fed to the current-carrying portion 34 to impose a voltage across the opposing electrodes E₃₁-E₃₂. The current-carrying method is, however, not limited to the above-described method, and any current-carrying method can be adopted insofar as a voltage can be imposed across the opposing electrodes E₃₁-E₃₂. In the hole 35, an unillustrated chucking mechanism is also inserted to hold or turn the bioassay plate 3.

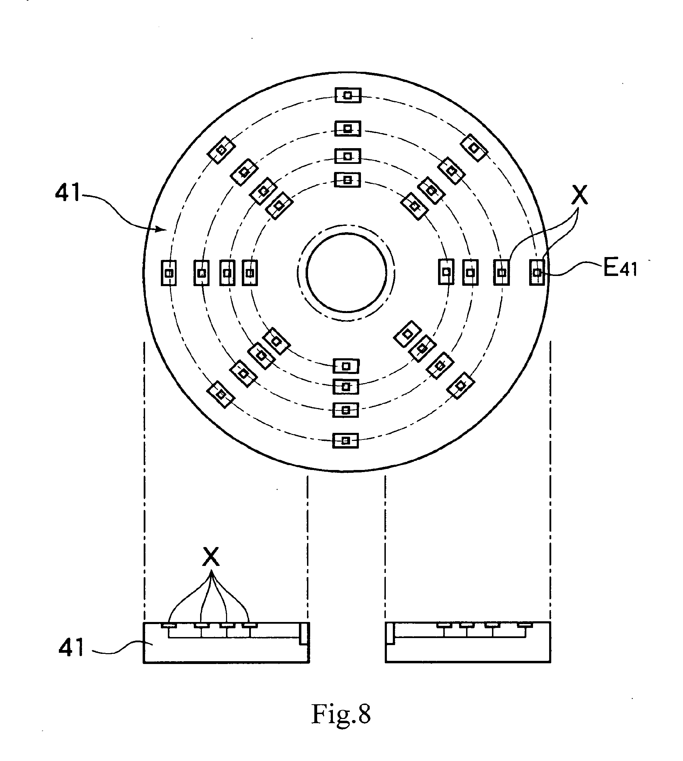

FIG. 8 is a view illustrating the construction of a modification of the disk-shaped first substrate which can be adopted in the bioassay substrate according to the present invention. A first substrate 41 as the illustrated modification is provided with a group of numerous detecting portions X arranged in a spiral pattern as viewed in top plan. Each detecting portion X is provided with a first electrode indicated at sign E₄₁ (see FIG. 8).

In the case of the group of detecting portions X of such an arrayed construction as in the first substrate 41, opposing electrodes can be also formed by stacking it with a second substrate (not shown) provided with a common second electrode arranged extending in a spiral form along the same track as the detecting portions X or arraying the above-described group of detecting portions in a spiral pattern or radial pattern and then stacking it, for example, with the above-described second substrate 32 or the like.

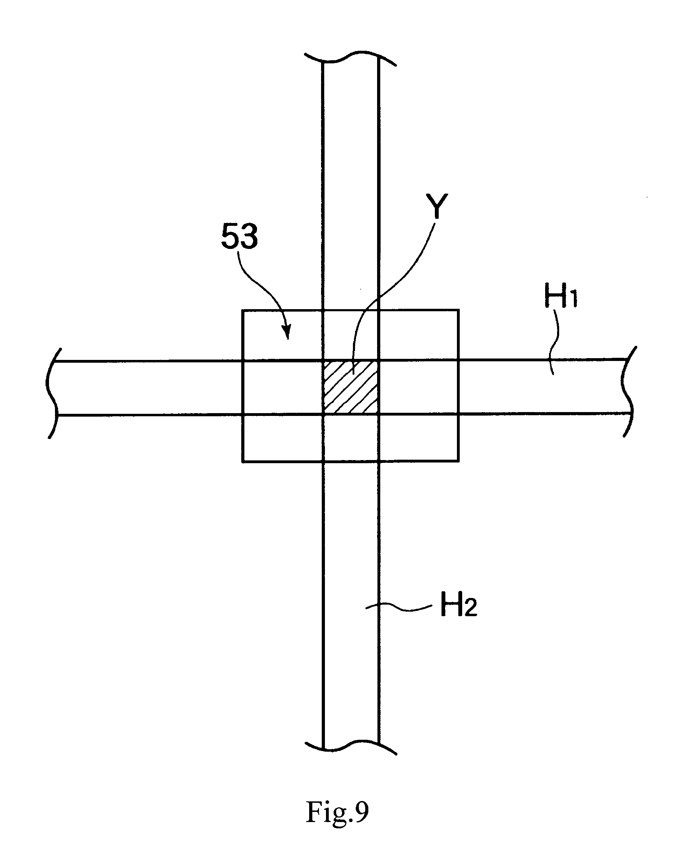

FIG. 9 is a view depicting another embodiment which can be adopted when forming opposing electrodes in the bioassay plate according to the present invention. The lower-side first substrate is provided with a current-carrying wiring (or an strip-shaped electrode) H1 such that the wiring extends transversely (or longitudinally) under a detecting portion X, while the upper-side second substrate is provided with a current-carrying wiring (or an strip-shaped electrode) H2 such that the wiring extends longitudinally (or transversely) over the detecting portion X. In such a construction as described above, opposing electrodes can be easily formed in a region Y where the current-carrying wirings (or strip-shaped electrodes) H1 and H2 intersect with each other.

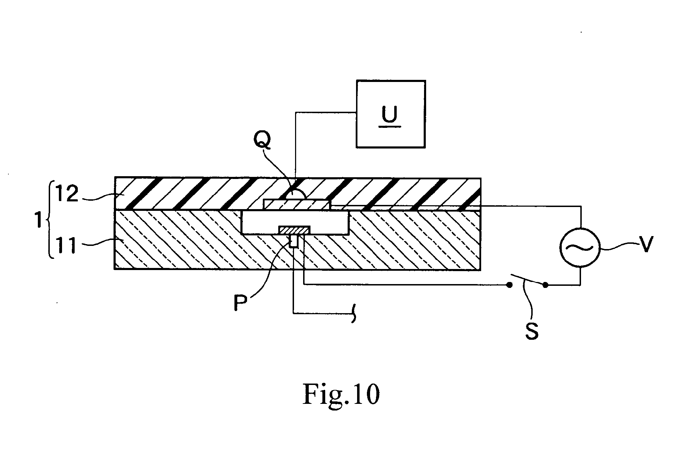

FIG. 10 is a view for describing one example of a developed form of the bioassay substrate according to the present invention. A description will be made of a case in which this embodiment is applied to the bioassay substrate 1 already mentioned above.

One of the electrodes making up opposing electrodes, that is, the electrode E₁₁ (or electrode E₁₂) is provided with a light source P of a very small size (for example, a light-emitting diode or semiconductor laser), the first substrate 11 is provided with a waveguide structure connected to the light source P via an optical fiber, and the other electrode E₁₂ (or electrode E₁₁) is integrally provided with a photodetector Q as a light-receiving portion for capturing fluorescence produced by emission light from the light source P (for example, fluorescence from a fluorescent intercalator).

In this case, the formation of each electrode E₁₁ or electrode E₁₂ with a transparent semiconductor such as ITO makes it possible to arrange the optical source (for example, light-emitting diode or semiconductor laser) P and photodetector Q inside the electrode E₁₁ or electrode E₁₂ or on a side not facing the reaction region R. It is to be noted that letter U in FIG. 10 indicates an analyzer unit connected to the photodetector Q.

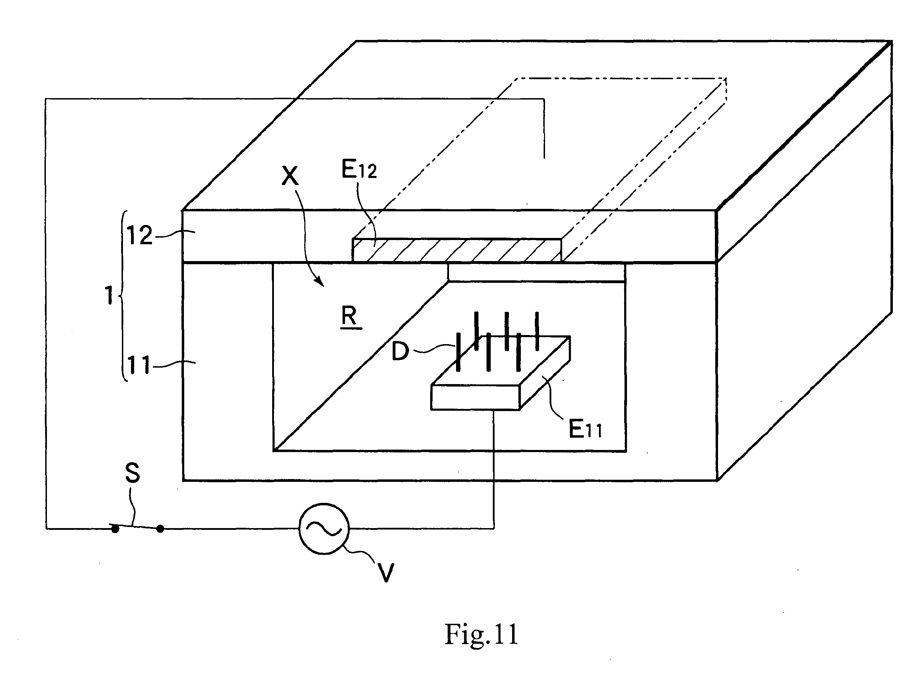

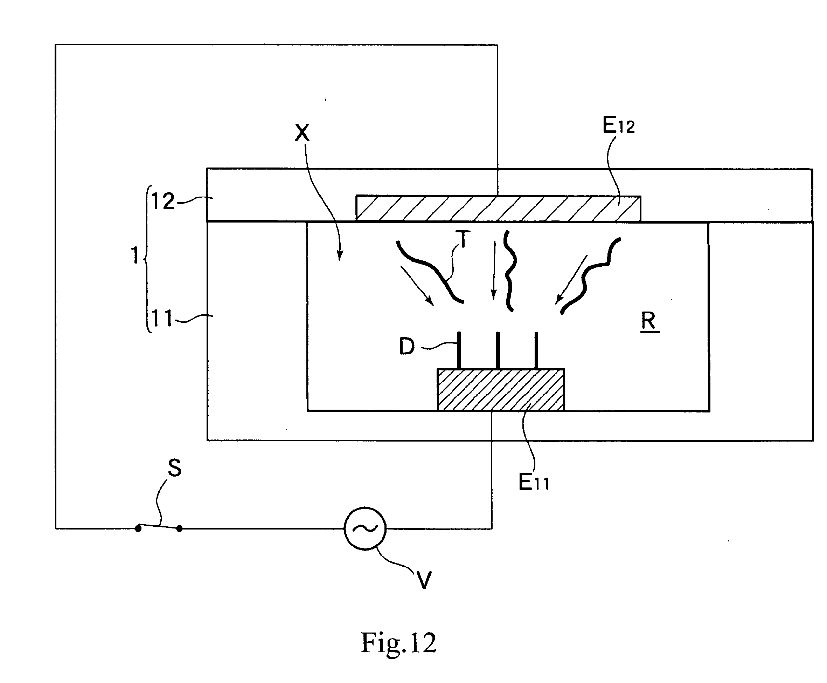

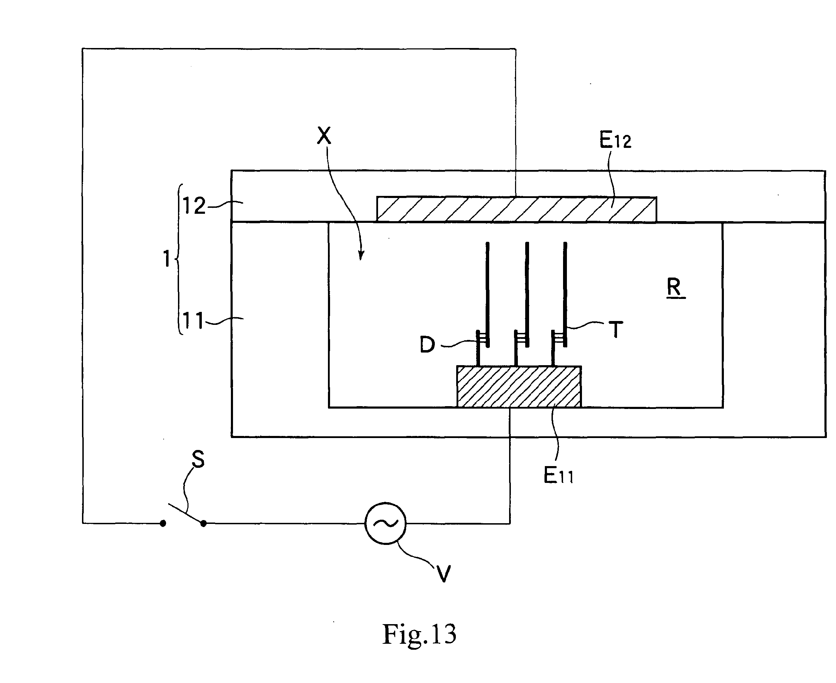

Using FIG. 11 through FIG. 13, a description will next be made of one example of a bioassay method which uses as a DNA chip the bioassay plate according to the present invention. In the following description on the method, the above-described bioassay plate of numeral 1 will be adopted as a representative example. FIG. 11 is a fragmentary, external, perspective view of the bioassay plate 1, and FIG. 12 and FIG. 13 are fragmentary, vertical, cross-sectional views.

### (Extension and immobilization of detecting substance)

Using an unillustrated inkjet nozzle, dispenser or the like, a sample solution which contains a detecting substance represented by a DNA probe D is dropped in a predetermined volume to the reaction region R which is in an open state.

Subsequently, the second substrate 12 is positioned on the first substrate 11 and accurately stacked with the first substrate 11. The switch S is then turned on to impose a high-frequency alternating electric field across the opposing electrodes E₁₁-E₁₂ via the power supply V. As the condition for the electric field imposed across the opposing electrodes E₁₁-E₁₂ at this time, an electric field of a high-frequency high voltage of approximately 1 × 10⁶ V/m and approximately 1 MHz is suited (see Masao Washizu and Osamu Kurosawa: "Electrostatic Manipulation of DNA in Microfabricated Structures", IEEE Transaction of Industrial Application, Volume 26, No. 26, PP 1165-1172 (1990)).

It is to be noted that the dropping of the sample solution to the reaction region R can be performed by forming a predetermined perforation through the second substrate 12 at a position facing the reaction region R and dropping it through the perforation. With such a construction, it is no longer necessary to detach the second substrate 12 from the top of the first substrate 11 and to open the reaction region R whenever the sample solution is dropped.

When the above-described high-frequency alternating electric field is formed for the reaction region R, electric lines of force concentrate around the surface of the electrode E₁₁, which has been formed with a smaller surface area than the electrode E₁₂ (see FIG. 11), or around an edge of the electrode E₁₁, so that a non-uniform electric field is formed in the reaction region.

Under the action of this non-uniform electric field, the DNA probe D as a detecting substance, which exists in a randomly-dispersed form in the reaction region R, extends in a direction along the non-uniform electric field and then migrates toward the electrode E₁₁ under the action of dielectrophoresis while being formed into a liner, high-order structure, and finally, molecule end portions of the detecting substance are immobilized through a specific coupling reaction or the like on the surface of the electrode E₁₂ (see FIG. 11). Subsequently, a predetermined buffer solution or the like may be poured into the reaction region R to perform its washing to remove the extra DNA probe D, and further, the reaction region R can be dried.

In particular, the surface of the first electrode E₁₁ to be used as a detecting surface on which the detecting substance is immobilized may be processed into the form of a rough surface containing concavities and convexities or asperities. When the surface of the first electrode E₁₁ is formed in such a pattern that concavities and convexities define islands, for example, electric lines of force are facilitated to concentrate at convex positions (land positions) on the surface of the first electrode E₁₁, thereby making it easier to form a non-uniform electric field and hence to align and immobilize the DNA probe D. No limitation is imposed on the form of the rough surface of the first electrode E₁₁. The processing of the electrode surface into the rough surface can be performed using, for example, a conventionally-known sputtering technique, etching technique or the like. However, no particular limitation is imposed on the processing technique.

### (Dropping and extension and migration of target substance T)

Next, a sample solution, which contains a target DNA (letter T) having a base sequence complementary with the immobilized DNA probe D, is dropped into the reaction region R which has been brought into an open state again by the detachment of the second substrate 12 (or is dropped through an unillustrated perforation arranged through the second substrate 12). Subsequently, the switch S is turned on to impose a high-frequency alternating electric field across the opposing electrodes E₁₁-E₁₂ via the power supply V. As the condition for the electric field imposed across the opposing electrodes E₁₁₋E₁₂ at this time, an electric field of a high-frequency high voltage of approximately 1 × 10⁶ V/m and approximately 1 MHz is suited (see Masao Washizu and Osamu Kurosawa: "Electrostatic Manipulation of DNA in Microfabricated Structures", IEEE Transaction of Industrial Application, Volume 26, No. 26, PP 1165-1172 (1990)).

By the imposition of the electric field, the target DNA indicated at letter T can be caused to migrate (move) toward the electrode E₁₁ of higher electric field intensity in the non-uniform electric field produced in the reaction region R under the electrodynamic effect of dielectrophoresis while causing it to extend (see FIG. 12) .

As a result, the target DNA indicated at letter T concentrates on the surface of the electrode E₁₁ where the DNA probe D designated at letter D has been immobilized beforehand, and the concentration of the target DNA becomes high there. An environment in which hybridization can proceed with ease is formed, thereby making it possible to shorten the hybridization time.

In addition, the DNA probe D can be aligned and immobilized in an extended state on the surface of the first electrode E₁₁ under the action of the non-uniform electric field. It is, therefore, possible to reduce the inhibition to hybridization due to the steric hindrance caused by the random-coiled high-order structure of the detecting substance or mishybridization. As a consequence, improvements can be made in the efficiency and accuracy of hybridization.

### (Hybridization)

Upon hybridization, the switch S is once turned off to bring the electric field into a state of zero in the reaction region R so that hybridization can be performed primarily by relying upon spontaneous Brownian motion. FIG. 13 schematically illustrates a state that hybridization has proceeded between the DNA probe D and the target DNA designated at letter T and as a result, a double stranded DNA has been formed.

The double-stranded DNA formed by the hybridization in the reaction region R emits fluorescence upon irradiation of excitation light predetermined for a fluorescent intercalator added to the reaction region R together with the target DNA designated at letter T or a fluorescent intercalator added to the reaction region R subsequent to the hybridization. By an optical means (spectroscopic means) for detecting the intensity of the fluorescence, the hybridization can be detected. The detection of the above-described hybridization can also be performed by a method that detects fluorescence emitted by a phosphor labeled on the DNA probe D as a detecting substance. It is to be noted that in the present invention, no particular limitation is imposed on the detection means.

### Industrial Applicability

According to the present invention, the efficiency of an interaction such as hybridization is high so that the time required for the interaction can be substantially shortened. In addition, the present invention can create an environment in which an accurate interaction is allowed to proceed with ease, and therefore, the occurrence of false positive or false negative can be controlled low. The present invention can, hence, be used to provide bioassay plates, such as DNA chips, equipped with characteristics that they are excellent in the efficiency of assay work for the detection of interactions and are also high in detection accuracy, and also to provide a method for their production.

## Claims

1. A bioassay plate comprising a first substrate and a second substrate stacked together,
said first substrate being provided at least with a reaction region for providing a place of an interaction between substances in a liquid phase and also with a first electrode arranged to face said reaction region, and
said second substrate being provided at least with a second electrode formed to be located oppose said first electrode.

2. The bioassay plate according to claim 1, wherein an axis of opposition between said first electrode and said second electrode is perpendicular to a bottom wall of said reaction region.

3. The bioassay plate according to claim 1, wherein a light source is arranged at a position of said first electrode, and a light-receiving portion is arranged at a position of said second electrode.

4. The bioassay plate according to claim 1, wherein a light-receiving portion is arranged at a position of said first electrode, and a light source is arranged at a position of said second electrode.

5. A method for producing a bioassay plate, which comprises providing:
a first substrate having a detecting portion provided at least with a reaction region for providing a place of an interaction between substances and also with a first electrode arranged to face said reaction region, and
a second substrate provided at least with a second electrode which permits imposition of an electric field to said reaction region in association with said first electrode; and
stacking these two substrates together such that said first electrode and said second electrode are located opposite each other.

6. The method according to claim 5, wherein a first substrate as defined in claim 5 and a second substrate as defined in claim 5 are stacked together, said first substrate carries thereon an array of plural detecting portions as defined in claim 5, and said second substrate is provided with a common second electrode for establishing a relation of opposing electrodes with first electrodes as defined in claim 5 and arranged in said respective plural detecting portions as a unit.

7. The method according to claim 5, wherein disk-shaped, said first substrate as defined in claim 5 and said second substrate as defined in claim 5 are stacked together, said first substrate carries thereon plural detecting portions as defined in claim 5 and arranged in a form of radial arrays from a center of said substrate, and said second substrate is provided with a strip-shaped or line-shaped, common second electrode as defined in claim 5 and formed extending over some or all of said detecting portions belonging to one of said radial arrays.

8. The method according to claim 5, wherein disk-shaped, said first substrate as defined in claim 5 and said second substrate as defined in claim 5 are stacked together, said first substrate carries thereon plural detecting portions as defined in claim 5 and arranged in a form of concentric arrays, and said second substrate is provided with a strip-shaped or line-shaped, common second electrode as defined in claim 5 and formed extending over some or all of said detecting portions belonging to one of said concentric arrays.

9. The method according to claim 5, wherein disk-shaped, said first substrate as defined in claim 5 and said second substrate as defined in claim 5 are stacked together, said first substrate carries thereon plural detecting portions as defined in claim 5 and arranged in a form of a spiral array, and said second substrate is provided with a strip-shaped or line-shaped, common second electrode as defined in claim 5 and formed extending over some or all of said detecting portions belonging to one of spiral array.

10. The method according to claim 5, wherein an axis of opposition between said first electrode and said second electrode is perpendicular to a bottom wall of said reaction region.

## Amended claims

### Amended claims under Art. 19.1 PCT

1. (Amended) A bioassay plate comprising a first substrate and a second substrate stacked together, said first substrate being provided at least with a reaction region for providing a place of an interaction between substances in a liquid phase and also with a first electrode arranged to face said reaction region, and
said second substrate being provided at least with a common second electrode formed to be located oppose said first electrode.

2. (Amended) The bioassay plate according to claim 1, wherein an axis of opposition between said first electrode and said common second electrode is perpendicular to a bottom wall of said reaction region.

3. (Amended) The bioassay plate according to claim 1, wherein a light source is arranged at a position of said first electrode, and a light-receiving portion is arranged at a position of said common second electrode.

4. (Amended) The bioassay plate according to claim 1, wherein a light-receiving portion is arranged at a position of said first electrode, and a light source is arranged at a position of said common second electrode.

5. A method for producing a bioassay plate, which comprises providing:
a first substrate having a detecting portion provided at least with a reaction region for providing a place of an interaction between substances and also with a first electrode arranged to face said reaction region, and
a second substrate provided at least with a second electrode which permits imposition of an electric field to said reaction region in association with said first electrode; and
stacking these two substrates together such that said first electrode and said second electrode are located opposite each other.

6. The method according to claim 5, wherein a first substrate as defined in claim 5 and a second substrate as defined in claim 5 are stacked together, said first substrate carries thereon an array of plural detecting portions as defined in claim 5, and said second substrate is provided with a common second electrode for establishing a relation of opposing electrodes with first electrodes as defined in claim 5 and arranged in said respective plural detecting portions as a unit.

7. The method according to claim 5, wherein disk-shaped, said first substrate as defined in claim 5 and said second substrate as defined in claim 5 are stacked together, said first substrate carries thereon plural detecting portions as defined in claim 5 and arranged in a form of radial arrays from a center of said substrate, and said second substrate is provided with a strip-shaped or line-shaped, common second electrode as defined in claim 5 and formed extending over some or all of said detecting portions belonging to one of said radial arrays.

8. The method according to claim 5, wherein disk-shaped, said first substrate as defined in claim 5 and said second substrate as defined in claim 5 are stacked together, said first substrate carries thereon plural detecting portions as defined in claim 5 and arranged in a form of concentric arrays, and said second substrate is provided with a strip-shaped or line-shaped, common second electrode as defined in claim 5 and formed extending over some or all of said detecting portions belonging to one of said concentric arrays.

9. The method according to claim 5, wherein disk-shaped, said first substrate as defined in claim 5 and said second substrate as defined in claim 5 are stacked together, said first substrate carries thereon plural detecting portions as defined in claim 5 and arranged in a form of a spiral array, and said second substrate is provided with a strip-shaped or line-shaped, common second electrode as defined in claim 5 and formed extending over some or all of said detecting portions belonging to one of spiral array.

10. The method according to claim 5, wherein an axis of opposition between said first electrode and said second electrode is perpendicular to a bottom wall of said reaction region.

11. (Added) The bioassay plate according to claim 1, wherein said first substrate as defined in claim 1 and said second substrate as defined in claim 1 are stacked together, said first substrate carries thereon plural detecting portions, and said second substrate is provided with a common second electrode for establishing a relation of opposing electrodes with first electrodes as defined in claim 1 and arranged in said respective plural detecting portions as a unit.

12. (Added) The bioassay plate according to claim 1, wherein disk-shaped, said first substrate as defined in claim 1 and said second substrate as defined in claim 1 are stacked together, said first substrate carries thereon plural detecting portions arranged in a form of radial arrays from a center of said substrate, and said second substrate is provided with a strip-shaped or line-shaped, common second electrode as defined in claim 1 and formed extending over some or all of said detecting portions belonging to one of said radial arrays.

13. (Added) The bioassay plate according to claim 1, wherein disk-shaped, said first substrate as defined in claim 1 and said second substrate as defined in claim 1 are stacked together, said first substrate carries thereon plural detecting portions arranged in a form of concentric arrays, and said second substrate is provided with a strip-shaped or line-shaped, said common second electrode as defined in claim 1 and formed extending over some or all of said detecting portions belonging to one of said concentric arrays.

14. (Added) The bioassay plate according to claim 1, wherein disk-shaped, said first substrate as defined in claim 1 and said second substrate as defined in claim 1 are stacked together, said first substrate carries thereon plural detecting portions arranged in a form of a spiral array, and said second substrate is provided with a strip-shaped or line-shaped, said common second electrode as defined in claim 1 and formed extending over some or all of said detecting portions belonging to one spiral array.

15. (Added) The bioassay plate according to claim 1, wherein said first electrode and said common second electrode are electrodes for forming an alternating electric field between said first electrode and said common second electrode.
